# EUROPEAN PATENT APPLICATION

(11) **EP 3 159 278 A1**
(43) Date of publication of application: **26.04.2017**
(21) Application number: 16195023.3
(22) Date of filing: 21.10.2016
(51) Int. Cl.: B65D 75/32

(54) **BLISTER FOR TIOTROPIUM BROMIDE INHALABLE FORMULATION**

(30) Priority: 23.10.2015 TR 201513289
(71) Applicant: Arven Ilac Sanayi Ve Ticaret A.S., Istanbul 34460 (TR)
(72) Inventor: TÜRKYILMAZ, Ali, 34460 Istanbul (TR); CELIK, Devrim, 34460 Istanbul (TR)
(74) Representative: Sevinç, Erkan

(57) **Abstract**

This invention is about a blister which is an integral component of a dry powder inhaler is structured from a base sheet and a lid sheet comprising at least two layers made from polymeric materials and flat aluminium foil, wherein the cavity that is used for storing the inhalable formulation is in the volume range of 32 to 60 mm³ and the cavity is filled up to 25-100 %.

## Description

### Field of Invention

This invention is about a blister which is an integral component of a dry powder inhaler is structured from a base sheet and a lid sheet comprising at least two layers made from polymeric materials and formed or flat aluminium folio, wherein cavity that is used for storing inhalable formulation is in the volume range of 32 to 60 mm³ and the cavity is filled up to 25-100 %.

### Background of Invention

Asthma and chronic obstructive pulmonary disease (COPD) affect more than 30 million people in the United States. More than 100,000 deaths each year are attributable to these conditions. Obstruction to airflow through the lungs is the characteristic feature in each of these airway diseases, and the medications utilized in treatment are often similar. Chronic obstructive pulmonary disease (COPD) is a widespread chronic lung disorder encompassing chronic bronchitis and emphysema. The causes of COPD are not fully understood. Experience shows that the most important cause of chronic bronchitis and emphysema is cigarette smoking. Air pollution and occupational exposures may also play a role, especially when combined with cigarette smoking. Heredity also causes some emphysema cases, due to alpha1 anti-trypsin deficiency.

Administration of asthma drugs by an oral inhalation route is very much in focus today, because of advantages offered like rapid and predictable onset of action, cost effectiveness and high level of comfort for the user. Dry powder inhalers (DPI) are especially interesting as an administration tool, compared to other inhalers, because of the flexibility they offer in terms of nominal dose range, i.e. the amount of active substance that can be administered in a single inhalation.

Dry powder inhalers (DPI) are becoming more and more popular because of their ease of use and medical efficacy. DPI's can be divided into two major categories: bulk and pre-metered devices. Pre-metered devices are gaining more and more market share due to the ability to control the product and process of metering a correct dose to the user. DPIs with pre-metered doses are, because of this, more reliable than bulk inhalers that meter the powder dose inside the inhaler. A pre-metered DPI moves the critical step of metering a dose to a pharmaceutical production process.

Anticholinergic agents, e.g. tiotropium, especially tiotropium bromide, are effective bronchodilators. These medicaments have a relatively fast onset and long duration of action, especially tiotropium bromide, which may be active for up to 24 hours. Anticholinergic agents reduce vagal cholinergic tone of the smooth muscle, which is the main reversible component of COPD. Anticholinergic agents have been shown to cause quite insignificant side effects in clinical testing, dryness of mouth and constipation are perhaps the most common symptoms. Because it is often very difficult to diagnose asthma and COPD correctly and since both disorders may co-exist, it is advantageous to treat patients suffering temporary or continuous bronchial obstruction resulting in dyspnoea with a small but efficient dose of a long-acting anticholinergic agent, preferably tiotropium bromide, because of the small adverse side effects.

Tiotropium bromide is known from European Patent Application EP 418 716 A1 and has the following chemical structure shown as Formula.I. Tiotropium bromide is the preferred anticholinergic agent because of its high potency and long duration. However, tiotropium is difficult to formulate in dry powder form to provide acceptable performance in terms of dose efficacy using prior art DPIs. Dose efficacy depends to a great deal on delivering a stable and high fine particle dose (FPD) out of the dry powder inhaler. It is also important to keep the dosage to the user as exact as possible and to maintain a stable efficacy over time, and that the medicament dose does not deteriorate during normal storage.

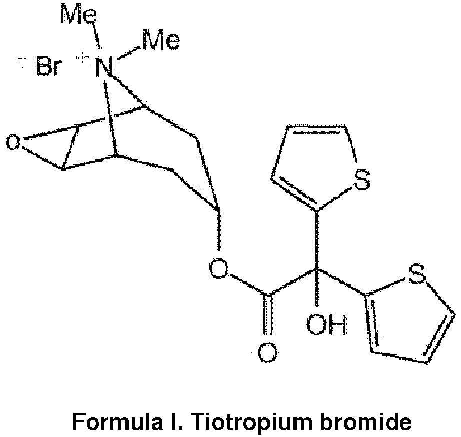

In prior art, methods of dose forming of tiotropium formulations include conventional mass, gravimetric or volumetric metering and devices and machine equipment well known to the pharmaceutical industry for filling blister packs, for example. Also see WO 03/27617 A1, WO 03/66437 A1, WO 03/66436 A1, WO 03/26965 A1, WO 02/44669 A1 and DE 100 46 127 A1, DE 202 09 156 U1 for examples of prior art in volumetric and/or mass methods and devices for producing doses of medicaments in powder form.

Electrostatic forming methods may also be used, for example as disclosed in US 6,007, 630 and US5,699,649.

A most suitable method of depositing microgram and milligram quantities of dry powders uses electric field technology (ELFID) as disclosed in U. S. Patent No. 6,592, 930 B2, which is hereby incorporated in this document in its entirety as a reference.

Preparing a formulation of tiotropium and an excipient where the amount of tiotropium is very small (e.g., < 1: 100 the amount of the excipient) is of utmost importance for the fine particle dose (FPD). Several prior art methods are aimed at improved preparation of excipients in order to improve the active ingredient fine particle dose (FPD) e.g. coating the excipient to present a fluorinated particle surface. Other surface modifications and surface treatment methods are possible to use to improve the fine particle dose (FPD) performance of the formulation.

In this present invention, a new technology is used for a blister. For this dry powder inhaler (DPI) which comprises tiotropium vacuum-drum technology is used. In this method, composition is vacuumed and filled into separate cavities. Drums are specialized for this inhalable powder characteristics comprising tiotropium. In prior art, powder particles are transported from a bulk source to a dose bed on the force of gravity or by using primarily electric and electrostatic force technology or by using membrane technology. One of the disadvantages of previous methods is only large amounts of powder can be filled (amount is about 13mg). Contrary to this, in the new technology of this invention, drums are specialized for the amount and characteristics and bulk density of the inhalable powder; so smaller amounts can be filled about 3-6 mg.

### Description of the invention

This invention is about a blister which is an integral component of a dry powder inhaler is structured from a base sheet and a lid sheet comprising at least two layers made from polymeric materials and formed or flat aluminium folio, wherein cavity that is used for storing inhalable formulation is in the volume range of 32 to 60 mm³ and the cavity is filled up to 25-100 %.

In a preferred embodiment, wherein the cavity is preferably in the volume range of 35 to 50 mm³, more preferably in the range of 40 to 45 mm³.

In another preferred embodiment, the cavity having the volume described above is filled preferably up to 30-100 %, more preferably up to 30-70 %, the most preferably up to 40-60 %.

According to this embodiment, the blister is in a strip form. And it comprises two sheets. The lid sheet and the base sheet of the blister strip, consists of at least two layers such as polymeric layer, aluminum foil.

According to the present invention, the polymeric materials used for making the polymeric layers which are present in both the base sheet and lid sheet are selected from the group consisting of polyethylene, polypropylene, polystyrene, polyolefin, polyamide, polyvinyl chloride, polyurethane, vinyl acetate, polybutylmethacrylate, copolymer of vinyl chloride, polyethylene terephthalate (PET), polyvinyl chloride (PVC), ortho-phthalic aldehyde (OPA) or mixtures thereof.

According to the invention, aluminium foil is used both in the lid sheet and in the base sheet of the blister strip to provide high humidity and gas protection. These layers must have the sufficient thickness which provides the protection for the stability of humidity sensitive dry powder formulation which is stored in blister cavity.

According to the embodiment, aluminium foil that is used in the lid sheet of the blister strip is in the thickness range of 5 to 40 µm, preferably of 10 to 30 µm.

In another embodiment, aluminium foil that is used in the base sheet of the blister strip is in the thickness range of 30 to 60 µm, preferably of 35 to 55 µm.

The polymeric layers which are contained in the lid sheet and the base sheet of the blister strip in accordance with the present invention may be made from either same of different polymers. The thickness of these polymeric layers depends on the type of polymeric substance used and its properties.

According to the embodiment, the polymeric layer which is used in the lid sheet of the blister strip is in the thickness range of 10 to 50 µm, preferably of 15 to 35 µm depending on the type of polymer used. The polymeric material used for making the polymeric layer is preferably polyethylene terephthalate (PET).

In another embodiment, the polymeric layer which is used in the base sheet of the blister strip is in the thickness range of 10 to 40 µm, preferably of 15 to 35 µm depending on the type of polymer used. The polymeric material used for making the polymeric layer is preferably ortho-phthalic aldehyde (OPA).

In one embodiment, the another polymeric layer which is used in the base sheet of the blister strip is in the thickness range of 80 to 120 µm, preferably of 90 to 110 µm depending on the type of polymer used. The polymeric material used for making the polymeric layer is preferably polyvinyl chloride (PVC).

Moreover, blisters which placed in the blister strips, can be in any shape wherein the polymeric layer forms a cavity molded.

The inside layer of blister cavity of said blister strip which is in contact with dry powder formulation is polymeric layer because of the fact that aluminium foil causes adhesion of part of dry powder formulation to inside layer of the cavity due to electrostatic forces, and hence cause uncontrolled dosing.

In one aspect, the present invention provides the dry powder formulation which contain tiotropium and is inhaled from the blisters. In another aspect, the present invention provides the dry powder formulation which contains tiotropium and inhaled by providing controlled dosing of the dry powder formulation in an effective and stable way.

In another aspect, the present invention provides the dry powder formulation which contains tiotropium and is inhaled in an effective, hygienic, user-friendly way. According to this invention, a blister which is an integral component of a dry powder inhaler wherein contains an inhalable formulation comprising tiotropium or an acceptable salt, solvate, hydrate thereof and at least one pharmaceutically acceptable excipient.

According to the invention, inhalable formulation comprising tiotropium or an acceptable salt, solvate, hydrate is tiotropium bromide.
In a preferred embodiment, the blister wherein contains an inhalable formulation comprising tiotropium bromide and at least one pharmaceutically acceptable excipient.

According to the invention, said dry powder formulation comprising tiotropium bromide in an amount in the range of 0.02 to 0.03 mg.

According to this embodiment, the amount of tiotropium bromide is present in an amount of 0.25 to 0.75 % by weight of total composition; preferably it is 0.35 to 0.45% by weight of total composition.

With active substances which have a particularly high efficacy, only small amounts of the active substance are needed per single dose to achieve the desired therapeutic effect. In such cases, the active substance has to be diluted with suitable excipients in order to prepare the inhalable powder. Because of the large amount of excipient, the properties of the inhalable powder are critically influenced by the choice of excipient. However, good flow properties are a prerequisite for highly accurate metering when packing and dividing up the individual doses of preparation, e. g. when producing capsules for powder inhalation or when the patient is metering the individual dose before using a multi-dose inhaler. The term inhalable proportion of active substance refers to the particles of the inhalable powder which are conveyed deep into the branches of the lungs when inhaled with a breath.

According to this invention, inhalable formulation comprising tiotropium includes at least one pharmaceutically acceptable excipient is selected from the group consisting of monosaccarides, disaccarides, polylactides, oligo-and polysaccarides, polyalcohols, glucose, arabinose, lactose, lactose monohydrate, lactose anhydrous, saccharose, maltose, dextrane, sorbitol, mannitol, xylitol, sodium chloride, calcium carbonate or mixtures thereof.

In a preferred embodiment, at least one pharmaceutically acceptable excipient is lactose monohydrate.

According to this embodiment, the amount of lactose monohydrate is present in an amount of 98.00 to 99.99 % by weight of total composition; preferably it is 99.00 to 99.99% by weight of total composition.

According to the invention, said dry powder formulation comprising lactose monohydrate in an amount in the range of 2.9 to 5.9 mg, preferably in an amount of 5.0 to 5.29 mg.

In this invention, a new technology is used. The process of filling cavities with inhalable formulation is vacuum-drum technology dose forming process. In this method, composition is vacuumed and filled into separate cavities. Drums are specialized for this inhalable powder characteristics comprising tiotropium. In prior art, powder particles are transported from a bulk source to a dose bed on the force of gravity or by using primarily electric and electrostatic force technology or by using membrane technology. One of the disadvantages of that methods is only large and constant amount of powder can be filled (amount is about 13mg). Contrary to this, in the new technology of this invention, drums are specialized for the amount and characteristics and bulk density of the inhalable powder; so smaller and changeable (according to the drums) amounts can be filled about 3-6 mg.

According to this embodiment, the cavity is filled about 3-6 mg of the formulation by is vacuum-drum technology dose forming process.

According to the invention preferably 5.3 mg of the aforementioned powder are delivered per dose.

Parameters of vacuum-drum device are approximately;
- Vacuum pressure: -50 - 400 mBar
- Blow pressure: 0.2 - 2.0 bar
- In-drum cleaning: 0.5 - 2.0 bar
- Dosing rate: 5-35 period/min
- Sealing temperature: 140-200 °C
- Compressive strength: 10-30 kN
- Sealing time: 200-600 ms

In this method powder flowability is less important, because powder particles are transported from a bulk source to a dose bed in a dose-forming step, not relying on the force of gravity but using vacuum-drum technology. An advantage of this vacuum-drum technology dose forming process is that it is not necessary to add large excipient particles to the medicament powder. Excipients are added to the active agent, particularly tiotropium, in order to dilute the drug to have a pre-metered dose in the inhaler.

### Example : Tiotropium bromide inhalable formulation

| **Ingredients** | **Amount (mg)** |
|---|---|
| tiotropium bromide | 0.0217 |
| lactose monohydrate | 5.2783 |

### Manufacturing Process:

- Lactose monohydrate is weighted into a glass container.
- The walls of shaker container are plastered with a sum of lactose monohydrate.
- Lactose monohydrate and Tiotropium bromide are weighed and added into the shaker container.
- A sum of lactose monohydrate is added into the shaker container and mixed for 3 minutes. This process is repeated two times more with lactose monohydrate again.
- Obtained mixture is sieved through 125 µm hand sieve to obtain a homogenous mixture without causing no particle size change.
- The whole mixture is taken into the same shaker container again.
- The sieve is cleaned with rest amount of lactose monohydrate.
- The mixture is mixed for 105 minutes with shaker container. In order to reduce electrostatic charge of the micronized active substance and to provide mixing homogenously, "electrostatic eliminator" is used during the process.

### Blister Filling Process:

Vacuum-drum technology dose forming process is used for blister filling. The blister cavity is in the volume range of 40 to 45 mm³ and the cavity is filled preferably up to 40-60 % by vacuum-drum technology dose forming process. The powder which is filled into the cavity is in the amount of 3-6 mg.

During the process parameters of vacuum-drum device are;
- Vacuum pressure: -50 - 400 mBar
- Blow pressure: 0.2 - 2.0 bar
- In-drum cleaning: 0.5 - 2.0 bar
- Dosing rate: 5-35 period/min
- Sealing temperature: 140-200 °C
- Compressive strength: 10-30 kN
- Sealing time: 200-600 ms

Finally, the blister strips are coiled into the Sanohaler medical device.

## Claims

1. A blister which is an integral component of a dry powder inhaler is structured from a base sheet and a lid sheet comprising at least two layers made from polymeric materials and formed or flat aluminium folio, wherein cavity that is used for storing inhalable formulation is in the volume range of 32 to 60 mm³ and the cavity is filled up to 25-100 %.

2. The blister according to claim 1, wherein the cavity is in the volume range of 35 to 50 mm³, preferably in the range of 40 to 45 mm³ and the cavity is filled up to 30-100 %, preferably up to 30-70 %, more preferably up to 40-60 %.

3. The blister according to claim 1, wherein the blister is in a strip form.

4. The blister according to claim 1, wherein the polymeric materials used for making the polymeric layers which are present in both the base sheet and lid sheet are selected from the group consisting of polyethylene, polypropylene, polystyrene, polyolefin, polyamide, polyvinyl chloride, polyurethane, vinyl acetate, polybutylmethacrylate, copolymer of vinyl chloride, polyethylene terephthalate (PET), polyvinyl chloride (PVC), ortho-phthalic aldehyde (OPA) or mixtures thereof.

5. The blister according to claim 1, wherein the aluminium foil that is used in the lid sheet of the blister strip is in the thickness range of 5 to 40 µm, preferably of 10 to 30 µm.

6. The blister according to claim 1, wherein the aluminium foil that is used in the base sheet of the blister strip is in the thickness range of 30 to 60 µm, preferably of 35 to 55 µm.

7. The blister according to claim 1, wherein the polymeric layer which is used in the lid sheet of the blister strip is in the thickness range of 10 to 50 µm, preferably of 15 to 35 µm.

8. The blister according to claim 4 and 7, wherein the polymeric material used for making the polymeric layer is preferably polyethylene terephthalate (PET).

9. The blister according to claim 1, wherein the polymeric layer which is used in the base sheet of the blister strip is in the thickness range of 10 to 40 µm, preferably of 15 to 35 µm.

10. The blister according to claim 4 and 9, wherein the polymeric material used for making the polymeric layer is preferably ortho-phthalic aldehyde (OPA).

11. The blister according to claim 1, further comprising another polymeric layer which is used in the base sheet of the blister strip is in the thickness range of 80 to 120 µm, preferably of 90 to 110 µm.

12. The blister according to claim 4 and 11, wherein the polymeric material used for making the polymeric layer is preferably polyvinyl chloride (PVC).

13. The blister according to any preceding claim, wherein comprising an inhalable formulation comprising tiotropium bromide and at least one pharmaceutically acceptable excipient.

14. The blister according to claim 13, wherein tiotropium bromide is present in an amount of 0.25 to 0.75 % by weight of total composition, preferably it is 0.35 to 0.45% by weight of total composition.

15. The blister according to claim 13, wherein at least one pharmaceutically acceptable excipient is lactose monohydrate.

16. The blister according to claim 17, wherein lactose monohydrate is present in an amount of 98.00 to 99.99 % by weight of total composition; preferably it is 99.00 to 99.99% by weight of total composition.

17. The blister according to any preceding claim, wherein the process of filling cavities with inhalable formulation is vacuum-drum technology dose forming process and the cavity is filled about 3-6 mg of the formulation by this process.
